(19) Europäisches Patentamt
European Patent Office
Office européen des brevets

(11) **EP 2 319 521 B1**

(12) **EUROPEAN PATENT SPECIFICATION**

(45) Date of publication and mention
of the grant of the patent:
**25.05.2016   Bulletin 2016/21**

(51) Int Cl.:
***A61K 36/732*** *(2006.01)*     ***A61P 19/02*** *(2006.01)*

(21) Application number: **09770384.7**

(22) Date of filing: **24.06.2009**

(86) International application number:
**PCT/KR2009/003403**

(87) International publication number:
**WO 2009/157712 (30.12.2009 Gazette 2009/53)**

(54) **CRUDE DRUG COMPOSITION FOR CARTILAGE REGENERATION, PAIN SUPPRESSION, AND EDEMA SUPPRESSION**

ROHE ARZNEIMITTELZUSAMMENSETZUNG ZUR REGENERATION VON KNORPEL, SCHMERZUNTERDRÜCKUNG UND ÖDEM-UNTERDRÜCKUNG

COMPOSITION MÉDICAMENTEUSE BRUTE POUR RÉGÉNÉRATION CARTILAGINEUSE, SOULAGEMENT DES DOULEURS ET SUPPRESSION DES OEDÈMES

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **24.06.2008   KR 20080059627**

(43) Date of publication of application:
**11.05.2011   Bulletin 2011/19**

(73) Proprietor: **Viromed Co., Ltd.**
**Seoul 151-747 (KR)**

(72) Inventors:
• **KIM, Sun Young**
**Pocheon-si**
**Gyeonggi-do 487-821 (KR)**
• **KIM, Seon-Hee**
**Kyoung-Ki-Do 427-741 (KR)**
• **PARK, Su Jin**
**Seoul 150-754 (KR)**
• **EO, Hae Kwan**
**Seongnam-si**
**Gyeonggi-do 463-920 (KR)**

• **CHOI, Jin Yong**
**Hwaseong-si**
**Gyeonggi-do 445-960 (KR)**
• **CHO, Byung Wook**
**Seoul 135-270 (KR)**

(74) Representative: **Towler, Philip Dean**
**Dehns**
**St Bride's House**
**10 Salisbury Square**
**London EC4Y 8JD (GB)**

(56) References cited:
WO-A1-03/059370          WO-A1-2004/090088
KR-A- 20010 084 485      KR-A- 20050 047 579

• **DATABASE WPI Week 200473 Thomson Scientific, London, GB; AN 2004-744777 XP002675919, & KR 2004 0060226 A (SHIM I S) 6 July 2004 (2004-07-06)**
• **KOREAN JOURNAL OF ORIENTAL PHYSIOLOGY AND PATHOLOGY vol. 21, no. 6, 2007, pages 1483 - 1490, XP008164177**
• **KOREAN JOURNAL OF ORIENTAL PHYSIOLOGY AND PATHOLOGY vol. 21, no. 2, 2007, pages 425 - 431, XP008164178**

**Description**

FIELD OF THE INVENTION

The present disclosure relates to a pharmaceutical composition which comprises herbal extracts of *Chaenomelis Fructus, Achyranthis Radix, Acanthopanax, Cinnamomi Cortex, Gentianae Radix, Clematidis Radix, Angelicae Radix, Cnidii Rhizoma, Gastrodiae Rhizoma, Safflower, Phlomidis Radix,* and *Ledebouriellae Radix,* wherein the extracts contain acanthoside D as active ingredient, for use in pain suppression, or edema suppression.

BACKGROUND OF THE INVENTION

Arthritis is largely classified into osteoarthritis (OA) and rheumatoid arthritis (RA).

Osteoarthritis, also known as degenerative arthritis, is the most common form of arthritis that afflicts middle-aged and elderly people. It causes pain, stiffness, and progressive motor dysfunction in the joints, e.g., knees and hips. Osteoarthritis involves in destruction of articular cartilage by matrix metalloproteinases (MMPs), which is characterized by degenerative changes in the articular cartilage and inflammation, leading to joint swelling and pain.

Rheumatoid arthritis is an inflammatory, autoimmune disease that affects multiple joints. Observed in arthritis patients are pathologies such as synovial tissue hyperplasia, subintimal infiltration of macrophages, dendritic cells, activated T and B lymphocytes, and accumulation of polymorphonuclear cells within the synovial fluid and on the surface of cartilage, inducing inflammation.

In addition, synovial tissue hyperplasia forms avascular pannus, a connective tissue layer occurring in articular cartilage, to cause pains due to irreversibly destroying the cartilage and bone, in which cartilage destruction factors such as MMPs are associated.

The ideal goal for treatment of osteoarthritis and rheumatoid arthritis is to protect and maintain cartilage and bone tissues and to ease the pain associated therewith.

Noxious stimuli evoking pain may be classified as mechanical, thermal, and chemical stimuli. These stimuli are perceived by nociceptors that are free nerve endings of pain-conducting $A\delta$ and C fibers, and intense and continuous stimuli lead to tissue damage and inflammation. Pathological pain results from tissue or nerve damages by noxious stimuli, inflammation, viral infection, and others.

Once inflammation takes place, immune cells or damaged cells produce and secrete proinflammatory cytokines such as interleukin-1 (e.g., IL-1$\alpha$, IL-1$\beta$, IL-6, tumor necrosis factor-$\alpha$ (TNF-$\alpha$), and others, as well as algesic mediators such as prostaglandin, which enhance pain sensitivity.

Conversely, inflamed tissues also secrete pain-suppressive opioid peptides such as endorphin, enkephalin, dynorphin, and others, or anti-inflammatory cytokines such as IL-4, IL-10, IL-13, and others, to exert analgesic effects.

Meanwhile, the knee cartilage covers the surface of the femur and tibia, which prevents the knee joint from abrasion by frictional force. But, in osteoarthritis, fibrosis and fissure occur on the surface of the cartilage, leading to damaged cartilage.

MMPs such as MMP-1, MMP-3, and MMP-13 play important roles in such metabolism of cartilage matrix, and inflammatory cytokines such as interleukin-1$\beta$ (IL-1 $\beta$), IL-6, TNF-$\alpha$) and other inflammatory mediators such as prostaglandin, nitrogen oxide (NO) are associated therewith.

MMPs are secreted from chondrocytes activated by mechnical stimuli, and degrade collagen and proteoglycan, main components of cartilage, and inflammatory cytokines and other inflammatory mediators not only promote the secretion of MMPs to enhance the MMPs' activities, but also stimulate inflammatory cells to aggravate inflammation.

MMPs' activities are regulated by specific tissue inhibitors of metalloproteinases (TIMPs), and therefore, up-regulation of TIMPs may inhibit MMPs' activities to exert beneficial effects. In addition, the increased anti-inflammatory cytokines including IL-4, IL-10, and IL-13 can also protect the cartilage matrix by inhibiting the production of inflammatory cytokines and promoting the production of TIMPs.

A number of therapeutic agents have been developed for the inhibition of inflammation and pain, but no drugs is currently available for cartilage regeneration. Further, as all of the foregoing drugs have limited effects, patients' severe pain as well as economical loss associated therewith still remain to be a subject to be resolved. At present, non-steroidal anti-inflammatory drugs (NSAIDs), gold-based drugs, penicillamine, and disease-modifying anti-rheumatic drugs (DMARDs) such as anti-malarials are used for the treatment of osteoarthritis and rheumatoid arthritis. However, these drugs have serious side effects.

Many arthritis therapeutics using natural products have been vigorously tested, but these are based on suppression of inflammatory reaction and have low curative effects. Thus, there is a need for the development of new arthritis therapeutics which has reduced side effects and excellent effects on the suppression of pain, edema, and cartilage regeneration.

The herbs used in the composition according to the present invention, including *Chaenomelis Fructus, Achy-*

*ranthis Radix, Acanthopanax, Cinnamomi Cortex, Gentianae Radix, Clematidis Radix, Angelicae Radix, Cnidii Rhizoma, Gastrodiae Rhizoma, Safflower, Rhlomidis Radix,* and *Ledebouriellae Radix,* are well-known as traditional herbal medicines which induce no significant adverse side effects, e.g., toxicity. The features and efficacies of these herbs are described below.

*Chaenomelis Fructus* is a dried from of the fruit of *Chaenomeles sinensis* KOEHNE or *Chaenomeles speciosa* NAKAI which belongs to Rosaceae, and has been used for treating acute gastroenteritis, beriberi, myalgia, arthritis, neuralgia, tussis, expectoration, pneumonia, and bronchitis (Park J. H. et al., The Encyclopedia of Medicinal Plants, 2000; Herbalogy, 1998).

*Achyranthis Radix* is the root of *Achyranthes japonica* (Miq.) NAKAI which belongs to Amaranthaceae and roots of *Achyranthes bidentata* BLUME and *Cyathula officinalis* KUAN are commercially available. It has been used for strengthening muscles and bones and protecting liver and kidney (Park J. H. et al., The Encyclopedia of Medicinal Plants, 2000; Herbalogy, 1998).

*Acanthopanax* is the root bark of *Acanthopanax gracilistylus* W. W. SMITH which belongs to Araliaceae, and it has been used for augmenting muscles and bones and treating edema (Park J. H. et al., The Encyclopedia of Medicinal Plants, 2000; Herbalogy, 1998).

*Cinnamomi Cortex* is the stem bark of *Cinnamomum cassia* BLUME which belongs to Lauraceae, and has been used for treating cold and ataralgesia (Modem Pharmacognosy, Hakchangsa, 1993).

*Gentianae Radix* is the root of *Gentiana macrophylla* PALLAS which belongs to Gentianceae, and has been used for treating paralysis (Park J. H. et al., The Encyclopedia of Medicinal Plants, 2000; Herbalogy, 1998).

*Clematidis Radix* is the root of *Clematis mandshurica* MAX. which belongs to Ranunculaceae, and has been used for treating gout (The Encyclopedia of Medicinal Plants, Park J. H. et al., Shinil Books, pp303-305, 2000; Herbalogy, Sanitation press, pp309-310, 1998).

*Angelicae Radix* is a dried form of the root of *Angelica gigas* NAKAI which belongs to Umbelliferae. Dried roots of *Angelica sinensis* DIELS and *Angelica acutiloba* KITAGAWA have been used in China and Japan, respectively, for puerperium disease, hematoporia, headache, and invigorating the blood stream (Park J. H. et al., The Encyclopedia of Medicinal Plants, 2000; Herbalogy, 1998).

*Cnidii Rhizoma* is the rhizome of *Cnidium officinale* MAKINO, *Ligusticum wallichii* FRANCH var. *officinale* YOOK, *Angelica genuflexa* MUTT of Japan, and *Ligusticum chuaxiong* HORT of China which belongs to Umbelliferae, and has been used for treating hematoporia, headache, obstetrics, and gynopathic disease (Park J. H. et al., The Encyclopedia of Medicinal Plants, 2000: Herbalogy, 1998).

*Gastrodiae Rhizoma* is a dried form of the rhizome bark of *Gastrodia elata* BLUME which belongs to Orchidaceae, and has been used for treating headache and dizziness (Park J. H. et al., The Encyclopedia of Medicinal Plants, 2000; Herbalogy, 1998).

*Safflower* is a dried form of the flower of *Carthamus tinctorius* L. which belongs to Compositae, and has been used for treating gynopathic disease, leukorrhea, and climacteric disease (The Encyclopedia of Medicinal Plants, Park J. H. et al. , Shinil Books, pp343-345, 2000; Herbalogy, Sanitation press, pp501-502, 1998).

*Phlomidis Radix* is the root of *Phlomis umbrosa* TURCZ which belongs to Labiatae, which has been used for treating bruise, fracture, and enhancing tissue regeneration (The Encyclopedia of Medicinal Plants, Park J. H. et al., 2000; Herbalogy, 1998).

*Ledebouriellae Radix* is the root of *Ledebouriella seseloides* WOLF which belongs to Umbelliferae, which has been used for treating cold, headache, arthritis, and tetanus (The Encyclopedia of Medicinal Plants, Park J. H. et al. , Shinil Books, pp160-161, 2000; Herbalogy, Sanitation press, pp169-171, 1998).

The herbs mentioned above have been used for acute and chronic inflammations such as various injuries, abscesses, pneumonia, bronchitis, anemia, headache, cold, and neuralgia, each having a unique pharmacological action and efficacy.

The complex herbal extract from above herbs (termed as "PG201") was found to be effective in protecting cartilage and preventing inflammation in a rat model of rheumatoid arthritis and a rabbit model of osteoarthritis (S. S. Shin, et al., J Rheumatol, 42, pp.665-672, 2003; KC Park, et al., Biochem Bioph Res Co, 331, pp.1469-1477, 2005). However, there has been no reported for either cartilage regeneration or pain suppression in clinical osteoarthritis models.

The present inventors have found unexpectedly that PG201 is effective in cartilage regeneration and pain and edema suppression via animal test.

<u>SUMMARY OF THE INVENTION</u>

Accordingly, it is an object of the present invention to provide a pharmaceutical composition which has no significant side effects, for use in pain suppression or edema suppression.

In accordance with one aspect of the present invention, there is provided a pharmaceutical composition which comprises herbal extracts of *Chaenomelis Fructus, Achyranthis Radix, Acanthopanax, Cinnamomi Cortex, Gentianae*

*Radix, Clematidis Radix, Angelicae Radix, Cnidii Rhizoma, Gastrodiae Rhizoma, Safflower, Phlomidis Radix,* and *Ledebouriellae Radix,* wherein the extracts contain acanthoside D as active ingredient, for use in pain suppression, or edema suppression.

Further, there is provided the above composition for use in pain suppression or edema suppression, in the form of a health food.

Still further, there is provided the above composition for use in pain suppression or edema suppression, in the form of a food or feed additive.

BRIEF DESCRIPTION OF DRAWINGS

The above and other objects and features of the present invention will become apparent from the following description of the invention, when taken in conjunction with the accompanying drawings.

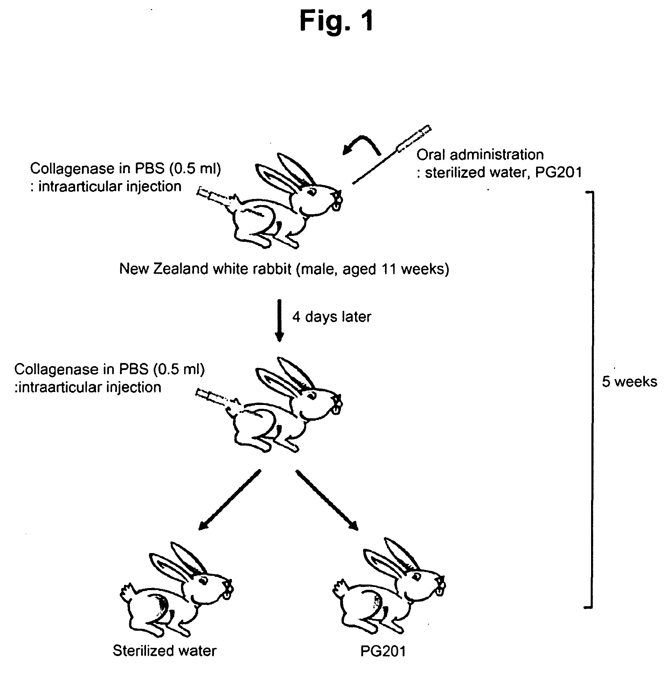

Figure 1: the schematic diagram showing the experiment for cartilage regeneration using collagen-induced arthritis in rabbits.

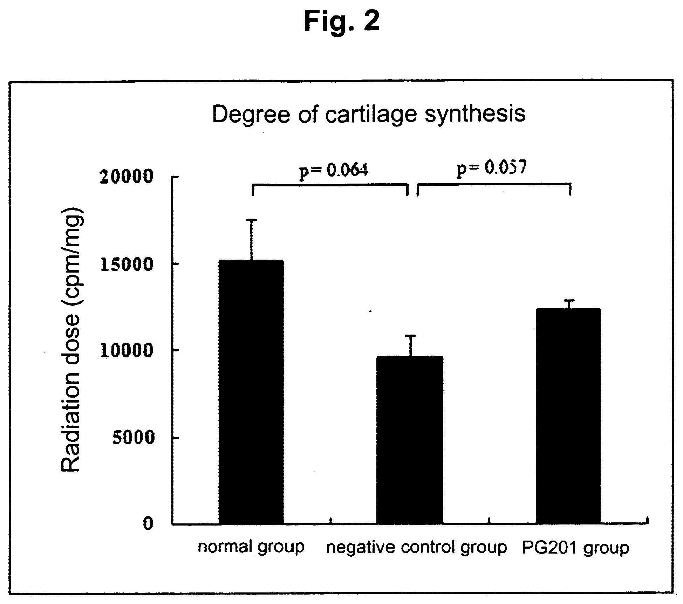

Figure 2: the effect of each experimental group on cartilage regeneration in collagen-induced arthritis in rabbits.

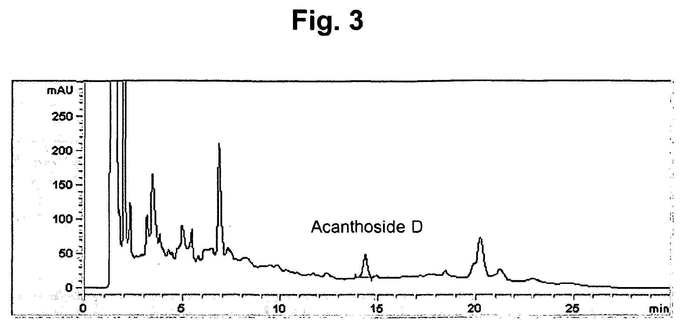

Figure 3: HPLC chromatogram showing acanthoside D in PG201.

DETAILED DESCRIPTION OF THE INVENTION

The pharmaceutical composition of the present invention may comprise the herbal extracts in an amount of 0.01 to 95% by weight, preferably 1 to 80% by weight, based on the total amount of the composition.

In addition, the inventive pharmaceutical composition may comprise extracts of *Chaenomelis Fructus, Achyranthis Radix, Acanthopanax, Cinnamomi Cortex, Gentianae Radix, Clematidis Radix, Angelicae Radix, Cnidii Rhizoma, Gastrodiae Rhizoma, Safflower, Rhlomidis Radix,* and *Ledebouriellae Radix,* wherein the weight ratios of the extracts are 1.5-2.5: 1.5-2.5: 1.5-2.5: 1.5-2.5: 0.75-1.75: 0.75-1.75: 0.75-1.75: 0.75-1.75: 0.75-1.75: 0.75-1.75: 0.5-1.5 : 1 , or may comprise an extract obtained from mixed herbs in the above ratios.

Further, said herbal extracts of the inventive pharmaceutical composition contain acanthoside D as active ingredient. The amount of acanthoside D may be 0.01~0.05% by weight, based on the total amount of the herbal extracts.

Also, the pharmaceutical composition of the present invention may further comprise other herbal extracts exerting same or similar effects.

The herbal extracts can be prepared by following procedure. Herbs are washed, and optionally dried, cut into small pieces, or pulverized. Then, the washed, dried, cut, or pulverized herbs are extracted in accordance with conventional extraction method, e.g., by using hot water or organic solvents. Further, the inventive composition may comprise a dried powder form derived from said extracts or a mixture of powders derived from commercially available extracts. In the preparation of herbal extracts, the herbs may be used in a dried or non-dried form, or a mixture thereof.

The herbal extracts of the inventive composition may be prepared, e.g., by cutting the dried herbs into small pieces, mixing, extracting the mixture 1 to 5 times in 1 to 20 folds of weight of water, organic solvent, or a mixed solvent thereof, preferably, 10 to 100% (v/v) of ethanol, for about 1 hour to 10 days, at 10°C to 100°C, in accordance with hot water extraction, cold water extraction, sonication, reflux or conventional extraction, and then filtering, concentrating, or freeze-drying the extract. Exemplary organic solvent includes $C_{1-4}$ low alcohols, ethylacetate, chloroform, hexane, dichloromethane, and others.

As shown in Experimental Examples of the present invention, the inventive pharmaceutical composition shows excellent *in vivo* cartilage regeneration in rabbit models of arthritis and significant pain inhibition in PBQ-induced writhing test (*see* Table 1). In addition, the composition shows excellent edema suppression in carrageenan-induced paw edema test (*see* Table 2).

The inventive pharmaceutical composition may further comprise pharmaceutically acceptable carriers, adjuvants, or diluents for use in treating arthritis or for use in pain suppression.

Examples of these carriers, adjuvants, or diluents include lactose, dextrose, sucrose, sorbitol, mannitol, xylitol, erythritol, maltitol, starches, acacia rubber, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate and mineral oil.

The inventive composition may be prepared in any form, e.g., oral dosage form such as a powder, granule, tablet, pill, capsule, extract, suspension, emulsion, syrup, aqueous medicine, aerosol, and others, topical preparation such as trast and patch, non-aqueous medicine, freeze-dried preparation, and sterile injectable preparation.

In the formulations, pharmaceutically acceptable fillers, extenders, binders, wetting agents, sweetening agents, flavoring agents, adjuvants, disintegrating agents, emulsifiers, lubricating agents and the like may be, if necessary, included.

The desirable dose of the inventive pharmaceutical composition may depend on the patient's age, sex or weight, but it may be generally administered 1 to 3 times per day in a divided dose of 0.01-10g, preferably, 1 to 5g. Further, the dose may be adjusted according to the administration route, severity of disease, patient's sex, weight, age and the like. Therefore, the dose does not limit the scope of the present invention in any way.

The present invention also provides a pharmaceutical composition which comprises herbal extracts of *Chaenomelis Fructus, Achyranthis Radix, Acanthopanax, Cinnamomi Cortex, Gentianae Radix, Clematidis Radix, Angelicae Radix, Cnidii Rhizoma, Gastrodiae Rhizoma, Safflower, Phlomidis Radix,* and *Ledebouriellae Radix,* wherein the extracts contain acanthoside D as active ingredient, for use in pain suppression, or edema suppression, in the form of a health food.

The health food of the present invention may comprise the herbal extracts in a total amount of 0.01 to 95% by weight, preferably 1 to 80% by weight.

Further, the weight ratio of the herbal extracts used in the inventive health food and preparation method thereof are aforementioned.

The health food of the present invention may be in any form of a powder, granule, tablet, capsule or beverage, and may further comprise other natural or synthetic substances for promoting health and additives for preparation.

The health beverage of the present invention may contain the herbal extracts in an amount of 1 to 30g, preferably 3 to 10g, based on 100 mL of beverage. Providing that the health beverage of present invention contains above described extracts as essential components in the indicated ratio, there is no particular limitation on the other liquid components, and the beverage may further contain various flavoring agents or natural carbohydrates which are conventionally used in beverages. Examples of aforementioned natural carbohydrates are monosaccharides such as glucose, fructose, and others; disaccharides such as maltose, sucrose, and others; conventional sugars such as dextrin, cyclodextrin, and others; and sugar alcohols such as xylitol, sorbitol, erythritol, and others. In addition to the carbohydrates, natural flavoring agents such as taumatin, stevia extract (e.g., levaudioside A, glycyrrhizin, etc.), and synthetic flavoring agents such as saccharin, aspartam, and others, may be used. The natural carbohydrates may be generally used in an amount of about 1 to 20g, preferably 5 to 12g per 100 mL of the composition.

Besides said components, the composition of the present invention may contain various nutritional supplements, vitamins, minerals (electrolytes), synthetic or natural flavoring agents, coloring agents, and fillers (cheese, chocolate, etc.), pectic acid and salts thereof, alginic acid and salts thereof, organic acids, protective colloid thickeners, pH adjusting agents, stabilizers, preservatives, glycerin, alcohols, carbonating agents used in carbonated beverages, and others. Further, the inventive composition may contain fruit pulps for natural fruit juices and fruit juice beverages and vegetable beverages. These components may be used independently or in combination. The ratio of such additives may be generally selected in a range of 0 to about 20 parts by weight based on 100 parts by weight of the composition.

Examples of the health food include various foods, beverages, gums, vitamin complexes, health food supplements, and others.

The present invention also provides a pharmaceutical composition which comprises herbal extracts of *Chaenomelis Fructus, Achyranthis Radix, Acanthopanax, Cinnamomi Cortex, Gentianae Radix, Clematidis Radix, Angelicae Radix, Cnidii Rhizoma, Gastrodiae Rhizoma, Safflower, Phlomidis Radix,* and *Ledebouriellae Radix,* wherein the extracts contain acanthoside D as active ingredient, for use in pain suppression, or edema suppression, in the form of a food or feed additive.

The food or feed additive of the present invention may comprise the herbal extracts in a total amount of 0.01 to 95% by weight, preferably 1 to 80% by weight based on the total amount of the composition.

Further, the weight ratio of the herbal extracts used in the inventive food or feed additive and preparation method thereof are aforementioned.

The additive may be added as a raw material on preparation of food or feed, or added to finally prepared food or feed.

The following Examples are intended to further illustrate the present invention.

**Example 1: Preparation of herbal extracts (PG201) using polar solvent (1)**

Twelve herbs, *Chaenomelis Fructus, Achyranthis Radix, Acanthopanax, Cinnamomi Cortex, Gentianae Radix, Clematidis Radix, Angelicae Radix, Cnidii Rhizoma, Gastrodiae Rhizoma, Safflower, Rhlomidis Radix,* and *Ledebouriellae Radix* were purchased from a market specializing in herbs (Kyungdong herb market, Seoul, Korea), and each herb was washed and dried. Then, 62.3g of *Chaenomelis Fructus,* 62.3g of *Achyranthis Radix,* 62.3g of *Acanthopanax,* 62.3g of *Cinnamomi Cortex,* 38.9g of *Gentianae Radix,* 38.9g of *Clematidis Radix,* 38.9g of *Angelicae Radix,* 38.9g of *Cnidii Rhizoma,* 38.9g of *Gastrodiae Rhizoma,* 38.9g of *Safflower,* 31.1g of *Rhlomidis Radix,* and 31.1g of *Ledebouriellae Radix* were mixed with 1.6L of 25% ethanol, and the mixture was extracted at a room temperature for 3 days while stirring. The extract was concentrated under a reduced pressure at 55~65°C, and freeze-dried to obtain an extract powder (PG201).

**Example 2: Preparation of herbal extracts (PG201) using polar solvent (2)**

Twelve herbs, *Chaenomelis Fructus, Achyranthis Radix, Acanthopanax, Cinnamomi Cortex, Gentianae Radix, Clematidis Radix, Angelicae Radix, Cnidii Rhizoma, Gastrodiae Rhizoma, Safflower, Rhlomidis Radix,* and *Ledebouriellae Radix* were washed and dried. Then, 1.0~1.5 L of 25% ethanol was added to 62.3g of *Chaenomelis Fructus,* 62.3g of *Achyranthis Radix,* 62.3g of *Acanthopanax,* 62.3g of *Cinnamomi Vortex,* 38.9g of *Gentianae Radix,* 38.9g of *Clematidis Radix,* 38.9g of *Angelicae Radix,* 38.9g of *Cnidii Rhizoma,* 38.9g of *Gastrodiae Rhizoma,* 38.9g of *Safflower,* 31.1 g of *Rhlomidis Radix,* and 31.1g of *Ledebouriellae Radix,* respectively, and each mixture was extracted at a room temperature for 3 days while stirring. The each extract was concentrated under a reduced pressure at 55~65°C, freeze-dried, and mixed to obtain an extract powder (PG201).

**Experimental Example 2: Pain inhibition effect of PG201 in PBQ-induced writhing test**

In order to measure pain inhibition effect of PG201, PBQ (para-benzoquinone)-induced writhing test was carried out. Male SD rats of 6 weeks age were orally administered with sterilized water (negative control group), with 400 mg/kg of PG201 (PG201 group), with 30 mg/kg of diclofenac sodium (Sigma, USA), a non-steroidal anti-inflammatory drug, as a positive control group (diclofenac group), and with 400 mg/kg of JOINS TAB (SK chemicals, Korea), a commercially available anti-inflammatory drug derived from natural substances (JOINS TAB group), respectively. Two hours after administration of test samples, the pain stimulus was induced in rats by intraperitoneal injection of 1 mg/kg of PBQ. The number of writhing by each rat was counted for 30 minutes from 10 minutes after injection of PBQ. Pain inhibition (%) was calculated from total number of writhes by using following equation 1 (Thuy, T. C. et al., J. Pharmacol Exp Ther. 248(3): pp907-915, 1989).

<Equation 1>

Pain inhibition (%) = [(number of writhes by negative control group – number of writhes by drug-treated group) / number of writhes by negative control group] × 100

As shown in Table 1, diclofenac group (positive control group) exhibited 92% of pain inhibition, and JOINS TAB group showed no pain inhibition. In contrast, PG201 showed 23% of pain inhibition, indicating that the inventive extracts are more effective in pain inhibition than previous natural therapeutic agents for arthritis.

<Table 1 >

| Experimental group | Diclofenac group | JOINS TAB group | PG201 group |
|---|---|---|---|
| Amount (mg/kg) | 30 | 400 | 400 |
| Percent inhibition of pain (%) | 92 | - | 23 |

**Experimental Example 3: Edema inhibition effect of PG201 in carrageenan-induced paw edema test**

In order to evaluate the effect of PG201 on edema inhibition, carrageenan-induced paw edema test was carried out. Male SD rats of 6 weeks age were orally administered with sterilized water (negative control group), with 400 mg/kg of PG201 (PG201 group), with 30 mg/kg of diclofenac as a positive control group (diclofenac group), and with 400 mg/kg of JOINS TAB (SK chemicals, Korea) (JOINS TAB group), respectively. Two hours after oral administration, 50 $\mu$L of 1% carrageenan solution was intradermally injected into the right hind paw. Immediately after injection and three hours after injection, the volumes of the hind paws were measured using a mercury displacement method. Edema inhibition (%) was calculated by comparing the mean edema of drug-treated group with that of negative control group (P. F. Moore et al., Inflamm. Res. (45) pp54-61, 1996).

As shown in Table 2, diclofenac group (positive control group) exhibited 92% of edema inhibition, and JOINS TAB group showed no edema inhibition. In contrast, PG201 showed 12% of edema inhibition, indicating the inventive extracts are more effective in edema inhibition than previous natural therapeutic agents for arthritis.

EP 2 319 521 B1

<Table 2>

| Experimental group | Diclofenac group | JOINS TAB. group | PG201 group |
|---|---|---|---|
| Amount (mg/kg) | 30 | 400 | 400 |
| Percent inhibition of edema (%) | 38 | - | 12 |

**Experimental Example 4: Analysis of amount of acanthoside D in PG201**

1.0 g of PG201 and 20 mL of 50% methanol were added to 50 mL of Mass flask, sonicated for 30 minutes, and centrifuged at 3,200 rpm, at a room temperature, for 30 minutes. The supernatant was filtered through 0.45 $\mu$m syringe filter, and the filtrate was used as a test solution. 10 mg of Standard acanthoside D was dissolved in methanol to make 100 mL, filtered through 0.45 $\mu$m syringe filter, and this solution was used as a standard solution. By subjecting 20 $\mu$L of the standard solution and 20 $\mu$L of the test solution to liquid chromatography under following conditions, the peak areas of acanthoside D, AS & AT, were measured, respectively. The amount of acanthoside D was calculated by using AS and AT from following equation.

$$\text{Equation: amount of acanthoside D } (C_{35}H_{60}O_6: 742.73) \text{ (mg)}$$
$$= \text{amount of standard acanthoside D (mg)} \times (AT / AS) \times$$
$$\text{purity of the standard}$$

Condition:

Detector: UV spectrophotometer (measurement wavelength 210 nm)
Column: Capcell Pak C18 UG80 4.6*150 (5 $\mu$M)
Temperature: 40°C
Mobile phase: A = 10% acetonitrile, B = 60% acetonitrile
Injection volume: 20 $\mu$L
Flow rate: 1 mL/min

<Table 3>

| Time (min) | A (%) | B (%) |
|---|---|---|
| 0 | 100 | 0 |
| 3 | 95 | 5 |
| 10 | 95 | 5 |
| 15 | 90 | 10 |
| 20 | 90 | 10 |
| 25 | 100 | 0 |
| 30 | 100 | 0 |

The amounts of acanthoside D in PG201 samples measured by HPLC quantification are shown in Table 4.

<Table 4>

| Sample No. of PG201 | Amount of acanthoside D (%) |
|---|---|
| 1 | n. d |
| 2 | 0.0139 |
| 3 | 0.0237 |
| 4 | 0.0360 |

EP 2 319 521 B1

(continued)

| Sample No. of PG201 | Amount of acanthoside D (%) |
|---|---|
| 5 | 0.0419 |

The following Preparation Examples are intended to further illustrate said pharmaceutical composition.

Preparation Example 1: Preparation of concentrated liquid

Herbal extract of Example 1    600 mg
Oligosaccharide    600 mg
Pear concentrate    500 mg
Vitamin mixture    30 mg
Strawberry flavor    appropriate amount

Concentrated liquid preparation was prepared by dissolving above components in distilled water to make 30 mL, filling the mixture in polyethylene ample and sterilizing.

Preparation Example 2: Preparation of tablet

Herbal extract of Example 1    300 mg
Lactose    85 mg
Corn starch    110 mg
Microcrystalline cellulose    63 mg
CMC-Ca    appropriate amount
Sodium starch glycolate    appropriate amount
Silicon dioxide    appropriate amount
Magnesium stearate    appropriate amount
or
Herbal extract of Example 1    300 mg
Potassium polyacrylate    80 mg
Hydroxypropyl cellulose    40 mg
Light anhydrous silicic acid    160 mg
Glyceryl behenate    appropriate amount
Magnesium stearate    appropriate amount

Tablet preparation was prepared by mixing above components, compressing and coating, in accordance with conventional tablet preparation method.

Preparation Example 3: Preparation of soft capsule

Herbal extract of Example 1    300 mg
Soybean oil    480 mg
Palm oil    90 mg
Yellow beeswax    30 mg

Soft capsule preparation was prepared by mixing above components and filling succinylated gelatin capsule by conventional soft capsule preparation method.

Preparation Example 4: Preparation of hard capsule

Herbal extract of Example 1    300 mg
Lactose    85 mg
Corn starch    110 mg
Microcrystalline cellulose    63 mg
CMC-Ca    10 mg

8

Hard capsule preparation was prepared by mixing above components and filling gelatin capsule by conventional hard capsule preparation method.

Preparation Example 5: Preparation of patch

| | |
|---|---|
| Crude drug composition of Example | 150 mg |
| Glycol salicylate | 150 mg |
| L-menthol | 100 mg |
| Methyl parahydroxybenzoate | 28 mg |
| Glycyrrhizic acid | appropriate amount |
| Diphenhydramine salicylate | appropriate amount |

A patch (14 cm x 10 cm) was prepared by mixing above components in accordance with conventional patch preparation method.

Preparation Example 6: Preparation of health food

| | |
|---|---|
| Herbal extract of Example 1 | 150 mg |
| Lactose | 120 mg |
| Milk calcium | 30 mg |
| Vitamin D3 | 30 mg |
| Vitamin C | 20 mg |
| Green tea extract powder | appropriate amount |
| Calcium panthothenate | appropriate amount |
| L-cystine | appropriate amount |
| Xylitol | appropriate amount |
| Magnesium stearate | appropriate amount |

Preparation Example 7: Preparation of health beverage

| | |
|---|---|
| Herbal extract of Example 1 | 1000 mg |
| Citric acid | 1000 mg |
| Oligosaccharide | 100 g |
| Concentrate of prnus mume | 2 g |
| Taurine | 1 g |
| Distilled water | to 900 mL |

Health beverage preparation was prepared by mixing above components, stirring at 87°C for about 1 hour, filtering, and filling in 1000 mL container and sterilizing by conventional health beverage preparation method.

Preparation Example 8: Preparation of food additive

Food additive preparation was prepared by using the herbal extract of Example 1 by itself, or by mixing the herbal extract of Example 1 with any of lactose, microcrystalline, corn starch and dextrin in 1:1, stirring, heat-drying at 40~70°C for 3~9 hours or vacuum-drying at 40~60°C and 760 mmHg or drying using a fluid bed dryer or spray-drying.

Preparation Example 9: Preparation of feed additive

Feed additive preparation was prepared according to Preparation Example 8.

**Claims**

1. A pharmaceutical composition which comprises herbal extracts of *Chaenomelis Fructus, Achyranthis Radix, Acanthopanax, Cinnamomi Cortex, Gentianae Radix, Clematidis Radix, Angelicae Radix, Cnidii Rhizome, Gastrodiae Rhizoma, Safflower, Phlomidis Radix,* and *Ledebouriellae Radix,* wherein the extracts contain acanthoside D as active ingredient, for use in pain suppression or edema suppression.

2. The composition of claim 1 for use in pain suppression or edema suppression, in the form of a health food.

3. The composition of claim 1 for use in pain suppression or edema suppression, in the form of a food or feed additive.

4. The composition of any of claims 1 to 3 for use in pain suppression or edema suppression, wherein the total amount of the extracts is 0.01 to 95% by weight, based on the total amount of the composition.

5. The composition of any of claims 1 to 3 for use in pain suppression or edema suppression, wherein the weight ratios of the extracts of *Chaeyaomelis Fructus, Achyranthis Radix, Acanthopanax*, *Cinnamomi Cortex, Gentianae Radix, Clematidis Radix, Angelicae Radix, Cnidii Rhizome, Gastrodiae Rhizome, Safflower, Phlomidis Radix,* and *Ledebouriellae Radix* are 1.5~2.5: 1.5~2.5: 1.5~2.5: 1.5~2.5: 0.75~1.75: 0.75~1.75: 0.75~1.75: 0.75~1.75: 0.75~1.75: 0.75~1.75: 0.5~1.5 : 1.

6. The composition of any of claims 1 to 3 for use in pain suppression or edema suppression, wherein the amount of acanthoside D is 0.01~0.05% by weight, based on the total amount of the extracts.

7. The composition of claim 1 further comprising pharmaceutically acceptable carriers, adjuvants, or diluents for use in treating arthritis or for use in pain suppression.

8. The composition of claim 1 for use in pain suppression or edema suppression, wherein the extracts are solvent extracts or powder forms derived therefrom.

9. The composition of claim 1 for use in pain suppression or edema suppression, wherein the composition is formulated into any one form selected from the group consisting of a powder, granule, tablet, pill, capsule, extract, suspension, emulsion, syrup, aqueous medicine, aerosol, trast, patch, non-aqueous medicine, freeze dried preparation, and sterile injectable preparation.

10. The food of claim 2 for use in pain suppression or edema suppression, wherein the food is in the form of a powder, granule, tablet, capsule, or beverage.


**Patentansprüche**

1. Eine pharmazeutische Komposition, die pflanzliche Extrakte aus *Chaenomelis Fructus, Achyranthis Radix, Acanthopanax, Cinnamomi Cortex, Gentianae Radix, Clematidis Radix, Angelicae Radix, Cnidii Rhizoma, Gastrodiae Rhizoma, Saflor, Phlomidis Radix,* und *Ledebouriellae Radix* umfasst, **dadurch gekennzeichnet, dass** die Extrakte als aktive Ingredienz Acanthosid D zur Verwendung in der Schmerzbeseitigung und Ödemunterdrückung enthalten.

2. Die Komposition gemäß Anspruch 1 zur Verwendung in der Schmerzbeseitigung oder Ödemunterdrückung in der Form einer Reformkost.

3. Die Komposition gemäß Anspruch 1 zur Verwendung in der Schmerzbeseitigung oder Ödemunterdrückung in der Form eines Nahrungsmittel- oder Futtermittelzusatzes.

4. Die Komposition gemäß einem der Ansprüche 1 bis 3 zur Verwendung in der Schmerzbeseitigung oder Ödemunterdrückung, **dadurch gekennzeichnet, dass** die Gesamtmenge an Extrakten von 0,01 bis 95% Gewicht im Verhältnis zur Gesamtmenge der Komposition ausmacht.

5. Die Komposition gemäß einem der Ansprüche 1 bis 3 zur Verwendung in der Schmerzbeseitigung oder Ödemunterdrückung, **dadurch gekennzeichnet, dass** die Gewichtsverhältnisse der Extrakte aus *Chaenomelis Fructus, Achyranthis Radix, Acanthopanax, Cinnamomi Cortex, Gentianae Radix, Clematidis Radix, Angelicae Radix, Cnidü Rhizoma, Gastrodiae Rhizoma, Saflor, Phlomidis Radix,* und *Ledebouriellae Radix* 1,5~2,5: 1,5~2,5: 1,5~2,5: 1,5~2,5: 0,75~1,75: 0,75~1,75: 0,75~1,75: 0,75~1,75: 0,75~1,75: 0,75~1,75: 0,5~1.5 : 1 ausmachen.

6. Die Komposition gemäß einem der Ansprüche 1 bis 3 zur Verwendung in der Schmerzbeseitigung oder Ödemunterdrückung, **dadurch gekennzeichnet, dass** die Menge Acanthosid D 0,01~0,05% Gewicht im Verhältnis zur Gesamtmenge der Extrakte ausmacht.

7. Die Komposition gemäß Anspruch 1, **dadurch gekennzeichnet, dass** sie darüber hinaus pharmazeutisch akzeptable Träger, Hilfsstoffe oder Verdünnungsmittel zur Verwendung in der Behandlung von Arthritis oder zur Verwendung in der Schmerzbeseitigung umfasst.

8. Die Komposition gemäß Anspruch 1 zur Verwendung in der Schmerzbeseitigung oder Ödemunterdrückung, **dadurch gekennzeichnet, dass** die Extrakte Lösungsmittelextrakte oder davon abgeleitete Pulverformen sind.

9. Die Komposition gemäß Anspruch 1 zur Verwendung in der Schmerzbeseitigung oder Ödemunterdrückung, **dadurch gekennzeichnet, dass** die Komposition in einer beliebigen der nachfolgenden Formen formuliert wird, und zwar Pulver, Granulat, Tablette, Pille, Kapsel, Extrakt, Suspension, Emulsion, Sirup, wässrige Arznei, Aerosol, Trast, Pflaster, nicht-wässrige Arznei, Tiefkühlpräparat und steriles, injizierbares Präparat.

10. Das Nahrungsmittel gemäß Anspruch 2 zur Verwendung in der Schmerzbeseitigung oder Ödemunterdrückung, **dadurch gekennzeichnet, dass** das Nahrungsmittel die Form eines Pulvers, Granulats, einer Tablette, Kapsel oder eines Getränks hat.

**Revendications**

1. Composition pharmaceutique qui comprend des extraits d'herbes de *Chaenomelis Fructus, Achyranthis Radix, Acanthopanax, Cinnamomi Cortex, Gentianae Radix, Clematidis Radix, Angelicae Radix, Cnidii Rhizoma, Gastrodiae Rhizoma, Carthamus tinctorius, Phlomidis Radix* et *Ledebouriellae Radix,* dans laquelle les extraits contiennent de l'acanthoside D en tant qu'ingrédient actif, à utiliser dans la suppression de la douleur ou la suppression d'oedème.

2. Composition selon la revendication 1 à utiliser dans la suppression de la douleur ou la suppression d'oedème, sous la forme d'aliments naturels.

3. Composition selon la revendication 1 à utiliser dans la suppression de la douleur ou la suppression d'oedème, sous la forme d'un aliment ou d'un additif alimentaire.

4. Composition selon n'importe laquelle des revendications 1 à 3 à utiliser dans la suppression de la douleur ou la suppression d'oedème, dans laquelle la quantité totale des extraits est de 0,01 à 95 % en poids, sur la base de la quantité totale de la composition.

5. Composition selon n'importe laquelle des revendications 1 à 3 à utiliser dans la suppression de la douleur ou la suppression d'oedème, dans laquelle les rapports pondéraux des extraits de *Chaenomelis Fructus, Achyranthis Radix, Acanthopanax, Cinnamomi Cortex, Gentianae Radix, Clematidis Radix, Angelicae Radix, Cnidii Rhizoma, Gastrodiae Rhizoma, Carthamus tinctorius, Phlomidis Radix* et *Ledebouriellae Radix* sont 1,5 ~ 2.5 : 1,5 ~ 2,5 : 1,5 ~ 2,5 : 1,5 ~ 2,5 : 0,75 ~ 1,75 : 0,75 ~ 1,75 : 0,75 ~ 1,75 : 0,75 ~ 1,75 : 0,75 ~ 1,75 : 0,75 ~ 1,75 : 0,5 ~ 1,5 : 1.

6. Composition selon n'importe laquelle des revendications 1 à 3 à utiliser dans la suppression de la douleur ou la suppression d'oedème, dans laquelle la quantité d'acanthoside D est de 0,01 ~ 0,05 % en poids, sur la base de la quantité totale des extraits.

7. Composition selon la revendication 1, comprenant en outre des vecteurs, adjuvants, ou diluants acceptables d'un point de vue pharmaceutique à utiliser dans le traitement de l'arthrite ou à utiliser dans la suppression de la douleur.

8. Composition selon la revendication 1 à utiliser dans la suppression de la douleur ou la suppression d'oedème, dans laquelle les extraits sont des extraits de solvant ou des formes poudreuses obtenues de ceux-ci.

9. Composition selon la revendication 1 à utiliser dans la suppression de la douleur ou la suppression d'oedème, dans laquelle la composition est formulée dans n'importe quelle forme sélectionnée à partir du groupe constitué par une poudre, granule, comprimé, pilule, capsule, extrait, suspension, émulsion, sirop, médicament aqueux, aérosol, timbre Trast, timbre transdermique, médicament non aqueux, préparation lyophilisée et préparation injectable stérile.

10. Aliment selon la revendication 2 à utiliser dans la suppression de la douleur ou la suppression d'oedème, dans lequel l'aliment est sous la forme d'une poudre, granule, comprimé, capsule, ou boisson.

# Fig. 1

Collagenase in PBS (0.5 ml)
: intraarticular injection

Oral administration
: sterilized water, PG201

New Zealand white rabbit (male, aged 11 weeks)

4 days later

Collagenase in PBS (0.5 ml)
:intraarticular injection

5 weeks

Sterilized water                    PG201

# Fig. 2

# Fig. 3

Acanthoside D

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PARK J. H. et al.** The Encyclopedia of Medicinal Plants. 2000
- *Modem Pharmacognosy, Hakchangsa,* 1993
- **PARK J. H. et al.** The Encyclopedia of Medicinal Plants. Shinil Books, 2000, 303-305
- Herbalogy. Sanitation press, 1998, 309-310
- **PARK J. H. et al.** The Encyclopedia of Medicinal Plants. Shinil Books, 2000, 343-345
- Herbalogy. Sanitation press, 1998, 501-502

- **PARK J. H. et al.** The Encyclopedia of Medicinal Plants. Shinil Books, 2000, 160-161
- Herbalogy. Sanitation press, 1998, 169-171
- **S. S. SHIN et al.** *J Rheumatol,* 2003, vol. 42, 665-672
- **KC PARK et al.** *Biochem Bioph Res Co,* 2005, vol. 331, 1469-1477
- **THUY, T. C. et al.** *J. Pharmacol Exp Ther.,* 1989, vol. 248 (3), 907-915
- **P. F. MOORE et al.** *Inflamm. Res.,* 1996, vol. 45, 54-61